Europäisches Patentamt

European Patent Office

Office européen des brevets

(11) Publication number: **0 660 233 A2**

## EUROPEAN PATENT APPLICATION

(21) Application number: **94120452.1**

(22) Date of filing: **22.12.94**

(51) Int. Cl.⁶: **G06F 9/44**

(30) Priority: **23.12.93 US 178394**

(43) Date of publication of application:
**28.06.95 Bulletin 95/26**

(84) Designated Contracting States:
**CH DE FR GB IT LI**

(71) Applicant: **SPACE LABS MEDICAL, INC.**
**15220 Northeast 40th Street**
**Redmond,**
**Washington 98073 (US)**

(72) Inventor: **Smokoff, Timothy L.**

**7208-135th Place Southeast**
**Renton,**
**Washington 98059 (US)**
Inventor: **Taylor, Allen Keith**
**7301 Mine Shaft Road**
**Raleigh,**
**North Carolina 27615 (US)**

(74) Representative: **Patentanwälte Grünecker,**
**Kinkeldey, Stockmair & Partner**
**Maximilianstrasse 58**
**D-80538 München (DE)**

(54) Method and system for providing multilingual text in an electric medical device.

(57) A method and system for providing multilingual text in a electronic medical device is provided. In a preferred embodiment, a computer program contains language-generalized executable code for controlling the operation of a electronic medical device. The executable code displays a selected textual message in any of several available languages. The executable code first determines that the selected message should be displayed. Then, in response to determining that the selected message should be displayed, the executable code identifies a current language in which the selected message should be displayed from among several available languages. The executable code then retrieves the selected message expressed in the identified current language from a file containing the selected message expressed in each of the available languages, and displays the retrieved message.

FIG. 1
PRIOR ART

EP 0 660 233 A2

Technical Field

The invention relates generally to a method and system for providing textual messages, and, more specifically, to a method and system for providing multilingual text in a electronic medical device.

Background of the Invention

As electronic medical devices such as electronic heart monitors that monitor patients and deliver medical care to patients have become more sophisticated, they have been required to provide greater volumes of more complicated information to the medical personnel that use them and the service technicians that service them. These people to whom the information is provided are collectively known as users. Indeed, it is now relatively common for an electronic medical device to visually provide information to users in several forms at once. For instance, many electronic medical devices display quantitative data, such as data supplied by medical sensors, in graph form, while simultaneously displaying textual messages with contents that convey information like patient background information, warnings about patient condition, user instructions, and diagnostic messages.

Typically, displayed textual messages are based on standard messages stored in the electronic medical device. For example, an electronic medical device may display the following diagnostic message:

*"This device is out of service pending required maintenance."*

In order to display the diagnostic message, developers of an electronic medical device would include instructions in the program that controls the electronic medical device that correspond to the following pseudocode:

```
1      define MESSAGE61 "This device is out of service

2            pending required maintenance."

3      display MESSAGE61
```

The display statement on line 3 instructs the electronic medical device to display string MESSAGE61, defined in lines 1-2 to contain the text of the diagnostic message. This approach permits developers of the electronic medical device to specify the display of the diagnostic message at any other point in the program by simply repeating the contents of line 3.

A textual message displayed by an electronic medical device may also contain current information about the present state of the patient or of the electronic medical device. For example, an electronic medical device may display the following patient information message:

*"Patient admitted: 12/16/93"*

In the above example, the patient admission date, "12/16/93", is not available when the message text is originally written and stored in a message. Rather, the date is incorporated, from current values stored in variables maintained by the program, into the message as shown by the following pseudocode:

```
11     define MESSAGE71 "Patient admitted:   "

12     define MESSAGE72 "/"

13     display MESSAGE71, month, MESSAGE72, day,

14            MESSAGE72, year
```

The above pseudocode construct, called a compound message, combines the text of one ore more messages and the current value of variables like month, day, and year.

Originally, electronic medical devices were developed to display textual messages in a single language. As geographic markets expand for electronic medical devices, it becomes desirable to adapt them to display messages in different languages, called target languages. For example, it becomes desirable to adapt an electronic medical device originally developed to display messages in English to display messages in German. This is commonly done by electronic medical device developers by moving all of the message definitions for the program, such as those shown on lines 1-2, 12, and 13, into a separate program source file, called a message file. The following is a message file based on the foregoing examples:

```
31     define MESSAGE61 "This device is out of service
32          pending required maintenance."
33     define MESSAGE71 "Patient admitted:   "
34     define MESSAGE72 "/"
```

Because this file contains definitions of the messages expressed in English, it is called an English message file. The program instructions that remain after the messages are removed are stored in a file called the program source file. Foreign language translators are then engaged to translate the message strings in the message file into German. The translators produce the following German message file:

```
41     define MESSAGE61 "Diese Vorrichtung ist unbrauchbar
42          bis nach notvendig Aufrechterhaltung."
43     define MESSAGE71 "Patient eingeweist:   "
44     define MESSAGE72 "."
```

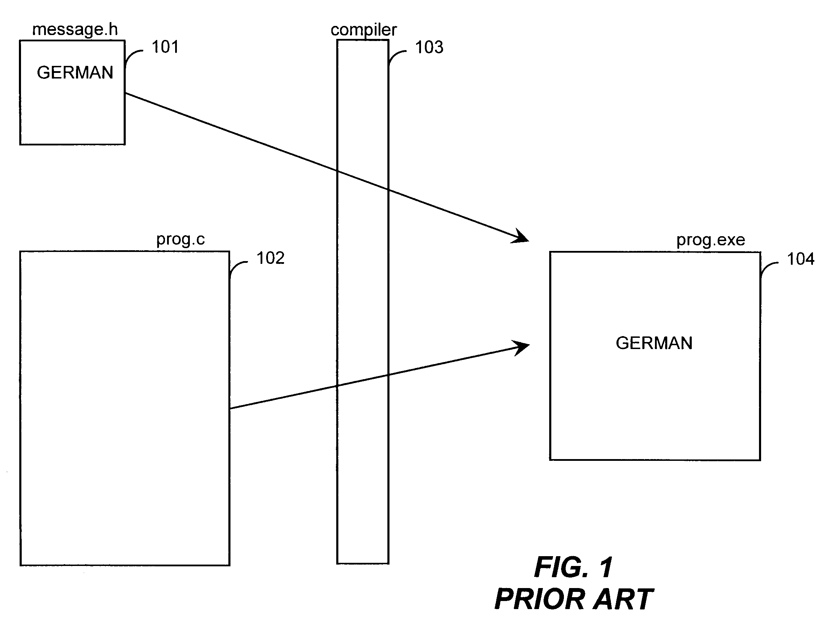

Figure 1 is a process diagram that shows a brief overview of the conventional combination of source code for the program with a language-specific message file during the program compilation process. The German message file 101, called "message.h", is submitted along with the program source file 102, called "prog.c", to the compiler 103. Given this input, the compiler produces a language-specific executable program 104 that can only display messages in one language, in this case German.

While the above approach does display messages in the target language, German, it has significant disadvantages. Foremost, the executable program produced can only display messages in a single language. A French-speaker using an electronic medical device with this program at a time when the electronic medical device needs service is only able to view the diagnostic message in German:

"*Diese Vorrichtung ist unbrauchbar bis nach notvendig Aufiechterhaltung.*"

In order for users to view messages in French, the program would have to be recompiled with a French message file, producing another language-specific executable program. Whenever a user wants to switch from French to German messages, the user must terminate the German language-specific executable program and start the French language-specific executable program, which is inconvenient. Also, because executable programs that control electronic medical devices are very large, storing a separate version of the executable program for each language used is very expensive in storage resource terms.

A further disadvantage of the above approach is that composite messages composed of defined messages and the current value of certain variables are not made available to translators in their entirety. That is, the translators are only permitted to translate each of the defined messages in isolation. This makes it impossible for some composite messages in which the order of variables must be altered by the translators to be translated correctly. For example, the following is the correct translation of the patient information message to German:

"*Patient eingeweist: 16.12.93*"

Note that the date is expressed in the form "day.month.year". Under the above approach, the German patient information message is displayed incorrectly, with the day and the month transposed, as follows:

"*Patient eingeweist: 12.16.93*"

As demonstrated above, the existing approach to providing message text in other languages in a electronic medical device has significant shortcomings: it cannot practicably provide messages in two or more different languages in the same electronic medical device, and it does not accommodate the correct translation of composite messages in which the order of variable values is different in different languages.

## Summary of the Invention

The invention provides a method and system for providing multilingual text in a electronic medical device. In a preferred embodiment, a computer program contains language-generalized executable code for controlling the operation of a electronic medical device The executable code displays a selected textual message in any of several available languages. The executable code first determines that the selected message should be displayed. Then, in response to determining that the selected message should be displayed, the executable code identifies a current language in which the selected message should be displayed from among several available languages. The executable code then retrieves the selected message expressed in the identified current language from a file containing the selected message expressed in each of the available languages, and displays the retrieved message.

Brief Description of the Drawings

Figure 1 is a process diagram that shows a brief overview of the conventional combination of source code for the program with a language-specific message file during the program compilation process.

Figure 2 is a process diagram that shows how the language-generalized program is produced.

Figure 3 is a high-level block diagram of the electronic medical device in which a preferred embodiment of the invention preferably operates.

Figure 4 is a flow diagram showing the steps necessary to develop a language-generalized program.

Figure 5 is a flow diagram showing the steps necessary to provide the message display service invoked by the language-generalized program to display a message.

Figure 6 is a flow diagram showing the steps required to perform the replacing step 503 shown in Figure 5.

Detailed Description of the Invention

A method and system for providing multilingual text in a electronic medical device is provided. In a preferred embodiment, a program comprised of code for controlling an electronic medical device, such as a heart monitor, is developed to incorporate steps that display multilingual text messages on a display device associated with the electronic medical device. The developed program (the program) is said to be language-generalized, and displays textual messages in any of several available languages. When the program is executing and reaches a point at which a particular message should be displayed, the program identifies a current language in which the message should be displayed from among several available languages. The program then retrieves the selected message expressed in the identified current language from a file containing the selected message expressed in each of the available languages, and displays the retrieved message.

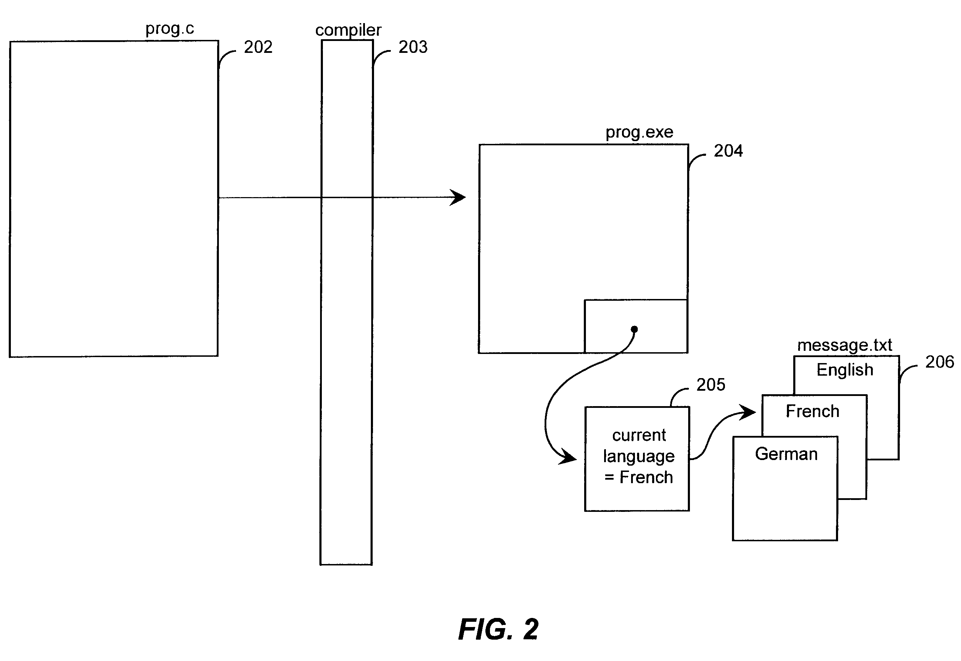

Figure 2 is a process diagram that shows how the language-generalized program is produced. A source file for the program 202, called "prog.c" is alone submitted to the compiler 203. Given this input, the compiler produces a language-generalized executable program 204. When the executable program needs to display a message, it uses a text display service provided according to the invention to check the value of a current language variable 205 set by the user using a user input device such as a touch-sensitive video monitor or keyboard. The text display service then retrieves the needed message in the current language from a message file 206 that contains messages in each of several available languages. It should be recognized that other storage schemes besides a file can be used. For example, messages may be stored in a database, or even inside the code for the language-generalized program.

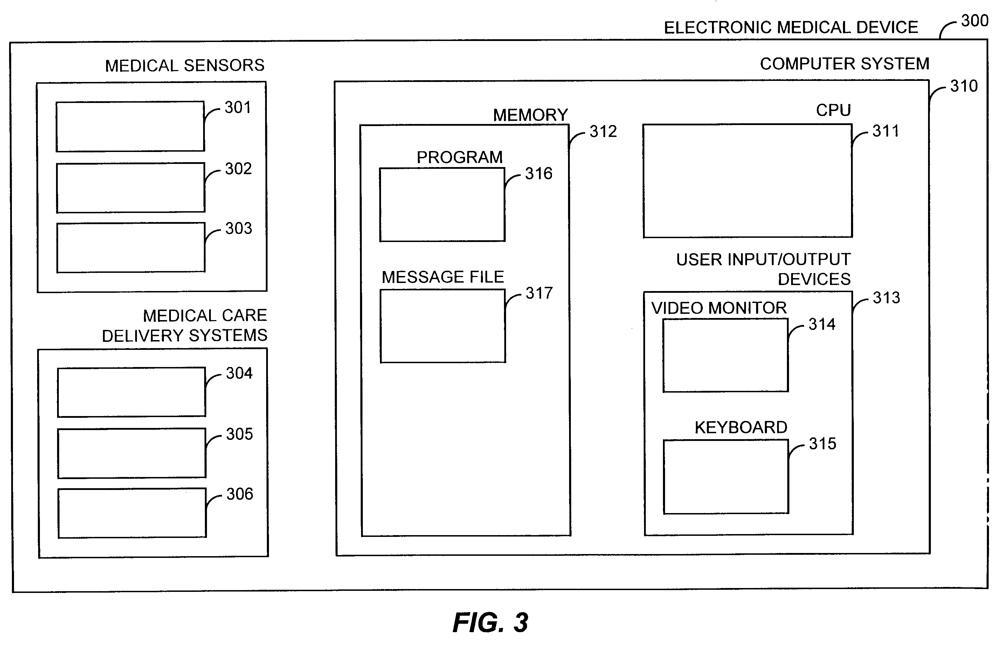

Figure 3 is a high-level block diagram of the electronic medical device in which a preferred embodiment of the invention operates. The electronic medical device 300 contains medical sensors 301-303, such as electrocardiac sensors, to monitor patients and medical care delivery systems 304-306, such as intravenous liquid delivery systems, to deliver medical care to patients. The electronic medical device also contains a computer system 310 to analyze data from the medical sensors, control the medical care delivery systems, and display information to and accept input from users. The computer system contains a central processing unit (CPU) 311, one or more computer memory devices (memory) 312, and user input/output devices 313 with which to display information, including messages, to and accept input from users. Among the user input/output devices is a video monitor 314, preferably a touch-sensitive video monitor, and a keyboard 315. The language-generalized program 316 and the message file 317 reside in a memory device. This memory device may be a solid-state memory device, a local disk drive, or even a remote disk drive controlled by a separate computer system to which the computer system 310 is connected via a network connection (not shown). The program executes on the CPU.

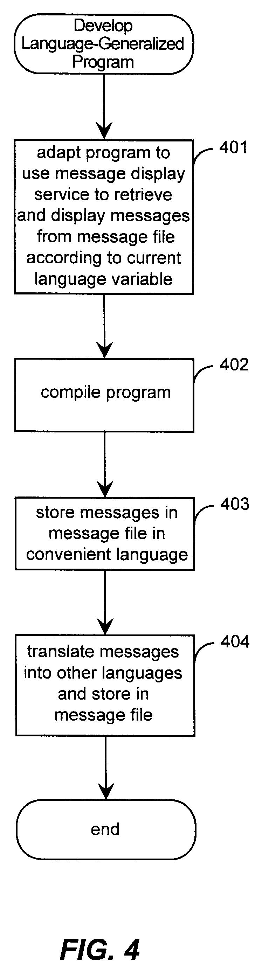

Figure 4 is a flow diagram showing the steps necessary to develop a language-generalized program. The steps shown in Figure 4 are preferably carried out by a human developer. In an alternate embodiment, automated developer tools carry out one or more of the steps. In step 401, the developer adapts a program for controlling a electronic medical device to use the message display service to display particular textual messages. For example, to cause the program to display the diagnostic message

*"This device is out of service pending required maintenance."*,

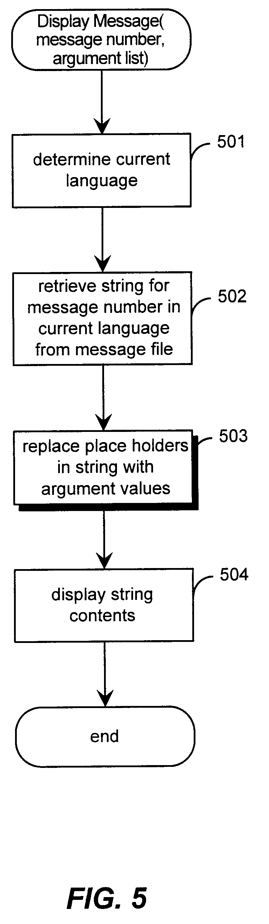

the developer includes an instruction in the program that invokes the message display service, described in more detail below in conjunction with Figure 5, to display a message having a particular reference number. The invocation is shown by the following pseudocode:

```
51      display message (91)
```

If the message to display incorporates the current value of any variables, these values are also passed to the message display service. For example, to display the following message

*"Patient admitted: 12/16/93"*

with correct admission date information, the developer would include an instruction corresponding to the following pseudocode, in which the values of variables admission month, admission day, and admission year are passed as an "argument list" to the message display service for inclusion in the message:

```
61      display message (92, admission month, admission day,
62              admission year)
```

In step 402, the developer compiles the program once. After step 402, the developer need never again recompile the program to enable it to display messages in other languages. In step 403, the developer defines the messages referenced in the program by storing their contents in a convenient language, here English, in the message file as follows:

| message number | English contents | German contents | French contents |
|---|---|---|---|
| ... | | | |
| 91 | "This device is out of service pending required maintenance." | | |
| 92 | "Patient admitted: %1d2/%2d2/%3d2" | | |
| ... | | | |

Note that the English contents of message number 92 contain three "place holders" containing the "%" character, specifically "%1d2", "%2d2", and "%3d2". Each place holder corresponds to a location in the message in which the current value of some variable should be inserted when the message is displayed. The number immediately after the "%" character designates the particular variable that is to be inserted, and refers to the order in which arguments are passed to the message display service. The "%1" place holder, for example, designates the first argument passed to the message display service. If the first argument is the admission month variable, as in line 61 above, then the "%1" place holder will be replaced with the current value of the admission month variable at the time of display. If the place holders occur in the message in the same order that the corresponding arguments appear in the argument list, the argument number may be omitted. Since this is the case in the English contents of message number 92, the English contents of message number 92 could merely be "Patient admitted: %d2/%d2/%d2" without altering the message displayed. The letter-numeral code at the end of the place holders (also optional) specifies the format in which the value of the argument should be displayed. Code "d2" means that the value should be displayed as a integer (truncated or expanded, if necessary) to two integral places. As further examples,

5

code "f2.1" means that the value should be displayed as a float to two integral places and one decimal place, and code "s10" means that the value should be displayed as a 10-character string. Besides a single variable, an argument can contain any expression that may be evaluated according to the rules of the programming language used. For example, if the variable internal temperature stores internal temperature in Kelvin and a message is to display internal temperate in degrees Celsius, the program would pass the parameter (internal temperature - 273).

In step 404, the developer or a specialized translator translates the messages in the file into other languages, then stores the translated messages in the file as follows:

| message number | English contents | German contents | French contents |
| --- | --- | --- | --- |
| ... | | | |
| 91 | "This device is out of service pending required maintenance." | "Diese Vorrichtung ist unbrauchbar bis nach notvendig Aufrechterhaltung." | "On ne se permis pas d'utiliser ce dispositif jusque ce que l'entretien necessaire est accompli." |
| 92 | "Patient admitted: %1d2/%2d2/%3d2" | "Patient eingeweist: %2d2.%1d2.%3d2" | "Le patient été admi: %2d2.%1d2.%3d2" |
| ... | | | |

Note that the date separators in the English contents of message number 92 ("/") are replaced with the separator most common in German (".") in the German contents. Further, the month and day arguments ("% 1d2" and "%2d2") are transposed in the German contents to comply with the European date ordering convention.

After step 404, these steps conclude. Step 404 may be repeated later at any time to permit the program to display message in still other languages. Further, the contents of any of the messages may be edited at a later time, and the language-generalized program may immediately display the new message contents without recompilation.

Figure 5 is a flow diagram showing the steps necessary to provide the message display service invoked by the language-generalized program to display a message. When the program invokes the message display service, it provides the message number of the message to be displayed, as well as an ordered argument list containing any variables whose value is to be incorporated in the message. See, for example, pseudocode lines 61-62 above.

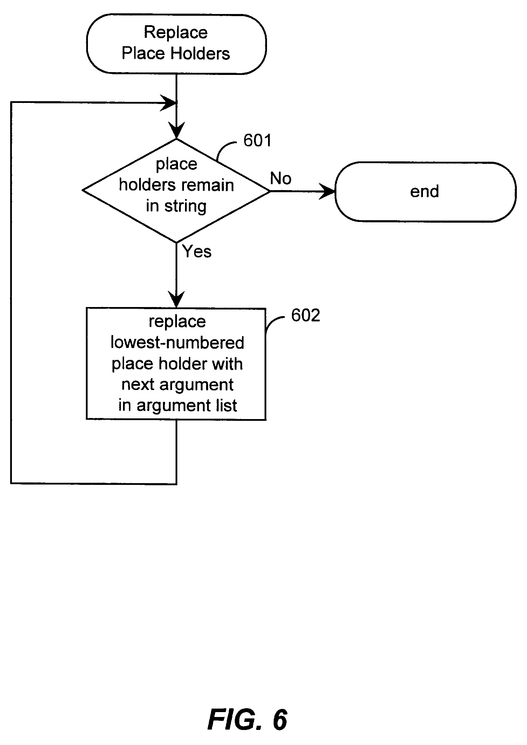

In step 501, the service determines the current language by checking the value of the current language variable 205. Because the value of the current language variable is checked each time a message is displayed, a user may change the value of the current language variable and have the change reflected in the very next message displayed. In step 502, the service retrieves from the file the string holding the message contents for the language corresponding the to value of the current language variable. In step 503, the service replaces any place holders in the retrieved message string with the values of the arguments passed to the service in the argument list. Step 503 is described in more detail below in conjunction with Figure 6. In step 504, the service displays the message string after any place holders have been replaced. Step 504 involves invoking a text display service provided by the programming language or operating system used and passing the message string to the text display service. These steps then conclude.

Figure 6 is a flow diagram showing the steps required to perform the replacing step 503 shown in Figure 5. In step 601, if any place holders remain in the retrieved message string, then the service continues at step 602, else these steps conclude and the service continues at step 504 to replace a place holder. In step 602, the service replaces the lowest numbered place holder in the string with the next argument in the argument list. The service then continues at step 601.

In a preferred embodiment, steps 501-502 are streamlined by maintaining a file descriptor containing information specifying the language in which a particular message should be retrieved. The file descriptor is modified to reflect the new value of the current language variable when the value of the current language variable is changed. The file descriptor can then be used in step 502 to retrieve the message in the language corresponding to the value of the current language variable without having to check the value of the current language variable in step 501.

While this invention has been shown and described with reference to preferred embodiments, it will be understood by those skilled in the art that various changes or modifications in form and detail may be made without departing from the scope of the invention.

**Claims**

1.  In a computer program containing language-generalized executable code for controlling the operation of a electronic medical device, a method performed by the executable code for displaying a selected textual message in any of a plurality of available languages, the method comprising the steps of:
    determining that the selected message should be displayed;
    in response to determining that the selected message should be displayed, identifying a current language in which the selected message should be displayed from among the plurality of available languages;
    retrieving the selected message expressed in the identified current language from a file containing the selected message expressed in each of the available languages; and
    displaying the retrieved message.

2.  The method of claim 1 wherein the file containing the selected message expressed in each of the available languages further contains a plurality of other messages expressed in each of the available languages, and wherein the selected message expressed in each of the available languages and each of the plurality of other messages expressed in each of the available languages are each distinguished by a unique identifier, and wherein the retrieving step includes the step of identifying message expressed in the determined current language having unique identifier of the selected message.

3.  The method of claim 2, further including the step of, before the step of determining that the selected message should be displayed, selecting the selected message from among the selected message and the other messages on the basis of a state of the computer program in which the message is to be displayed.

4.  The method of claim 1 wherein the executable code is used by a user, further including the step of permitting the user to alter the current language.

5.  In a computer program containing language-generalized executable code for controlling the operation of a electronic medical device, a method performed by the executable code for displaying one of a plurality of textual messages in any of a plurality of available languages, the method comprising the steps of:
    selecting a display language from among the plurality of available languages;
    selecting a display message for display from the plurality of textual messages, the display message reflecting the state of the computer program containing executable code;
    retrieving the display message in the display language from a file that contains substantially all of plurality of textual messages in substantially all of the plurality of available languages; and
    displaying the retrieved message.

6.  The method of claim 5 in which the step of selecting a display language from among the plurality of available languages selects a display language in response to an indication from a user of the program of which of the plurality of available languages to select.

7.  A method for developing an internationalized computer program containing language-generalized executable code for controlling the operation of a electronic medical device, the executable code being capable of displaying textual messages in two languages, the method comprising the steps of:
    storing text to be displayed by the program in a first language in a plurality of messages in a first language message file;
    translating the text in each of the plurality of messages in the first language message file from the first language to a second language;
    storing messages containing the text translated into the second language in a second language message file;
    including steps in the executable code that reserve space for, initialize, and permit a user to change the value of a current language variable that holds the identity of one of the two languages in

7

EP 0 660 233 A2

which text should be displayed; and

including text display steps in the executable code that cause the text in a particular message of the message file for the current language to be displayed.

8. The method of claim 7 wherein the executable code is capable of displaying textual messages in three languages, further including the steps of:

translating the text in each of the plurality of messages in the first language message file from the first language to a third language; and

storing messages containing the text translated into the third language in a third language message file;

and wherein the step of including steps in the executable code that reserve space for, initialize, and permit a user to change the value of a current language variable that holds the identity of one of the two languages in which text should be displayed includes the step of including steps in the executable code that reserve space for, initialize, and permit a user to change the value of a current language variable that holds the identity of one of the three languages in which text should be displayed.

9. In a computer program containing language-generalized executable code for controlling the operation of a electronic medical device, a method performed by the executable code for displaying a selected textual message in any of a plurality of available languages, the textual message containing a run-time value, the current language being any of the plurality of available languages, the method comprising the steps of:

determining that the selected textual message should be displayed;

in response to determining that the selected textual message should be displayed, determining the identity of a current language in which the selected message should be displayed;

retrieving the selected textual message expressed in the current language from a file containing the message expressed in each of the available languages, the selected textual message expressed in the current language containing a placeholder at an arbitrary position within the selected textual message expressed in the current language;

determining the current run-time value;

replacing the placeholder in the retrieved message with the current run-time value; and

displaying the retrieved message with the placeholder replaced by the current run-time value.

10. In a computer program containing language-generalized executable code for controlling the operation of a electronic medical device, a method performed by the executable code for displaying a selected textual message in any of a plurality of available languages, the textual message containing a plurality of run-time values, the method comprising the steps of:

determining that the selected message should be displayed;

in response to determining that the selected message should be displayed, identifying a current language in which the selected message should be displayed from among the available languages;

retrieving the selected message expressed in the identified current language from a file containing the selected message expressed in each of the available languages, the selected message expressed in the current language containing a plurality of place holders, each placeholder associated with a run-time value and at an arbitrary position within the selected message expressed in the current language;

while the retrieved message contains one or more place holders:

selecting a placeholder in the retrieved message,

determining the current run-time value with which the selected placeholder is associated, and

replacing the selected placeholder in the retrieved message with the current run-time value; and

displaying the retrieved message with the place holders replaced by the current run-time values with which the place holders are associated.

11. The method of claim 10 wherein the selected message expressed in the identified current language retrieved in the retrieving step contains the plurality of place holders occurring in a different order than the selected message expressed in a language other than the identified current language stored in the file containing the selected message expressed in each of the available languages.

12. An electronic medical device capable of displaying one of a plurality of textual messages in a plurality of available languages, the electronic medical device comprising:

a memory for storing:

8

a program for controlling the operation of the electronic medical device, therein selecting one of the plurality of textual messages to display,

the plurality of textual messages in a plurality of available languages, and

an indication of the available language in which to display textual messages;

a user input device for setting the indication of the available language in which to display textual messages to indicate that textual messages should be displayed in a particular available language; and

a display device for displaying the selected one of the plurality of textual messages in the language indicated by the indication of the available language in which to display textual messages.

13. The electronic medical device of claim 12 wherein the display device is a video monitor.

14. The electronic medical device of claim 12 wherein the user input device is a keyboard.

15. The electronic medical device of claim 12 wherein the user input device is a touch-sensitive video monitor.

16. The electronic medical device of claim 12, further including a processor for processing the program stored in the memory.

17. The electronic medical device of claim 16 wherein the memory further stores a program for inserting current information into the displayed textual message that is displayed on the display device, and wherein the processor processes the program for controlling the operation of the electronic medical device, therein selecting one of the plurality of textual messages to display and the program for inserting current information into the displayed textual message that is displayed on the display device, both stored in the memory.

18. The electronic medical device of claim 12 wherein the memory further stores a program for inserting current information into the displayed textual message that is displayed on the display device.

FIG. 1
PRIOR ART

FIG. 2

**FIG. 3**

ELECTRONIC MEDICAL DEVICE — 300

MEDICAL SENSORS
- 301
- 302
- 303

MEDICAL CARE DELIVERY SYSTEMS
- 304
- 305
- 306

COMPUTER SYSTEM — 310

MEMORY — 312
- PROGRAM — 316
- MESSAGE FILE — 317

CPU — 311

USER INPUT/OUTPUT DEVICES — 313
- VIDEO MONITOR — 314
- KEYBOARD — 315

EP 0 660 233 A2

Develop
Language-Generalized
Program

adapt program to
use message display
service to retrieve
and display messages
from message file
according to current
language variable — 401

compile program — 402

store messages in
message file in
convenient language — 403

translate messages
into other languages
and store in
message file — 404

end

**FIG. 4**

Display Message(
message number,
argument list)

determine current
language — 501

retrieve string for
message number in
current language
from message file — 502

replace place holders
in string with
argument values — 503

display string
contents — 504

end

*FIG. 5*

14

**FIG. 6**